# FASCICULE DE BREVET EUROPEEN

(11) **EP 1 706 330 B1**
(45) Date de publication et mention de la délivrance du brevet: **23.05.2007**
(21) Numéro de dépôt: 05717661.2
(22) Date de dépôt: 11.01.2005
(51) Int. Cl.: B65D 75/34, A61M 15/00

(54) **BANDE DE BLISTERS POUR INHALATEUR**
BLISTERSTREIFEN FÜR EINEN INHALATOR
BLISTER STRIP FOR AN INHALER

(30) Priorité: 14.01.2004 FR 0400313
(43) Date de publication de la demande: 04.10.2006
(73) Titulaire: Valois SAS, F - 27110 Le Neubourg (FR)
(72) Inventeur: QUONIAM, Michel, F-27320 La Madeleine de Nonancourt (FR)
(74) Mandataire: CAPRI
(86) Numéro de dépôt international: PCT/FR2005/050014
(87) Numéro de publication internationale: WO 2005/068317

(56) Documents cités:
- WO-A-03/090825
- US-A- 3 856 144
- US-A- 6 155 423
- US-A1- 2002 008 046

## Description

La présente invention concerne une bande de blisters, et plus particulièrement une bande de blisters destinée à être utilisée dans un inhalateur de produit fluide ou pulvérulent.

Les bandes de blisters sont généralement utilisées pour contenir de manière prédosée un produit, notamment un produit pharmaceutique, dans un appareil de distribution, tel qu'un inhalateur. Chaque blister forme ainsi un réservoir contenant une dose individuelle maintenue fermée de manière étanche avant son utilisation. Le dispositif comporte un moyen d'entraînement pour, à chaque actionnement, amener un blister dans une position lui permettant d'être ouvert, libérant ainsi la dose de produit qu'il contient, celle-ci étant ensuite distribuée à l'utilisateur. Les bandes de blisters de ce type comportent généralement une couche de cavités contenant le produit, et une couche déchirable ou pelable fixée sur la couche de cavités et permettant d'ouvrir successivement chaque blister. De telles bandes de blisters sont decrites dans les documents WO 03/090825 A1 et US 3 856 144. Plusieurs problèmes peuvent se poser avec ce type de bande de blisters. Ainsi, pour assurer une distribution de la totalité de dose de produit contenu dans le blister, l'ouverture doit se faire sur la totalité de la surface du blister, sans présenter de bavure ou partie résiduelle de couche déchirée restant fixée au blister, susceptible de retenir une partie de produit. Pour résoudre ce problème, les bandes qui sont ouvertes par pelage présentent un avantage par rapport aux bandes à déchirer. Par contre, un autre problème peut se poser, notamment lorsque la bande de blister est utilisée dans un inhalateur. En effet, à chaque actionnement de l'inhalateur, un seul blister doit être ouvert pour éviter tout risque de surdosage. En particulier avec des bandes qui sont ouvertes par pelage, il peut être difficile de garantir l'ouverture d'un seul blister à chaque actionnement de l'inhalateur, et généralement des moyens assez complexes doivent être mis en oeuvre pour assurer cette fonctionnalité. Un autre problème qui se pose avec les bandes pelables concerne le compromis qui doit être trouvé entre une bonne étanchéité des blisters lorsqu'ils sont fermés et une capacité d'ouverture qui doit permettre à l'appareil d'ouvrir un blister à chaque actionnement. Ce compromis est généralement assez difficile à trouver, et empêche généralement d'utiliser une ouverture du blister déclenchée par l'inhalation de l'utilisateur avec ce type de bande de blister.

La présente invention a pour but de fournir une bande de blister qui ne reproduit pas les inconvénients susmentionnés.

Plus particulièrement, la présente invention a pour but de fournir une bande de blister qui garantit une étanchéité absolue du produit contenu dans le blister avant son ouverture.

De plus, la présente invention a pour but de fournir une bande de blister qui assure une ouverture optimale de chaque blister, en garantissant une distribution de la totalité de la dose contenue dans chaque blister.

La présente invention a aussi pour but de fournir une bande de blister qui évite tout risque de surdosage.

La présente invention a encore pour but de fournir une bande de blisters qui peut être utilisée dans un inhalateur avec un système d'ouverture déclenché par l'inhalation de l'utilisateur.

La présente invention a aussi pour but de fournir une bande de blisters qui soit simple et peu coûteuse à fabriquer et à assembler.

La présente invention a donc pour objet une bande de blisters destinée à être utilisée dans un inhalateur de produit fluide ou pulvérulent, comportant une pluralité de blisters formés chacun par un réservoir comportant une ouverture obturée de manière étanche par une couche déchirable, caractérisé en ce que la bande de blisters comporte au moins une couche de base pourvue d'ouvertures formant les ouvertures des blisters, et une couche de cavités pourvue de cavités formant les parois de blisters, ladite couche déchirable comportant une première partie de couche déchirable disposée entre ladite couche de base et ladite couche de cavités, et une seconde partie de couche déchirable disposée du côté opposé de ladite couche de base, lesdites première et seconde parties de couche déchirable étant reliées l'une à l'autre au niveau de chaque ouverture de la couche de base.

Avantageusement, les première et seconde parties de couche déchirable sont réalisées avec le même matériau, avantageusement du polyéthylène, de préférence sous la forme d'un film continu de polyéthylène ayant une épaisseur inférieure à 100µm, avantageusement comprise entre 10µm et 40µm, de préférence 30µm.

Avantageusement, lesdites première et seconde parties de couche déchirable sont reliées l'une à l'autre de manière monobloc au niveau de chaque ouverture de la couche de base, de préférence par fusion de matière.

La couche de base comprend avantageusement du polyester, de préférence réalisée sous la forme d'un film ayant une épaisseur inférieure à 100 µm, avantageusement comprise entre 40µm et 60µm de préférence 50µm.

Avantageusement, ladite couche de cavités comprend du polyéthylène et/ou du polypropylène.

Avantageusement, la couche déchirable comporte en outre une première couche d'aluminium fixée à ladite seconde partie de couche déchirable, éventuellement avec interposition d'une couche de polyester et d'une couche d'adhésif, la couche d'aluminium ayant une épaisseur inférieure à 50µm, avantageusement comprise entre 10µm et 30µm, de préférence 20µm.

Une première couche externe, de préférence formée par une laque d'impression, peut également être prévue sur la couche déchirable.

La couche de cavités comporte avantageusement une seconde couche d'aluminium, éventuellement avec interposition d'une couche de polyester et d'une couche d'adhésif.

Avantageusement, ladite couche de cavités comporte une seconde couche externe, de préférence formée par une couche de protection ou une couche de vernie, de préférence avec interposition d'une couche d'adhésif.

Avantageusement, l'adhérence de la couche déchirable à la couche de base entre les ouvertures est différente de l'adhérence à proximité desdites ouvertures.

Avantageusement, les blisters contiennent une poudre pharmaceutique.

La présente invention a aussi pour objet un inhalateur de poudre sèche comportant une bande de blisters telle que décrite ci-dessus.

D'autres caractéristiques et avantages de la présente invention apparaîtront plus clairement au cours de la description détaillée suivante, faite en référence aux dessins joints, donnés à titre d'exemples non limitatifs, et sur lesquels :
la figure 1 est une vue schématique éclatée de la structure d'une bande de blisters selon un mode de réalisation avantageux de la présente invention, et
la figure 2 est une vue schématique en perspective d'une bande de blister en cours d'ouverture d'un blister.

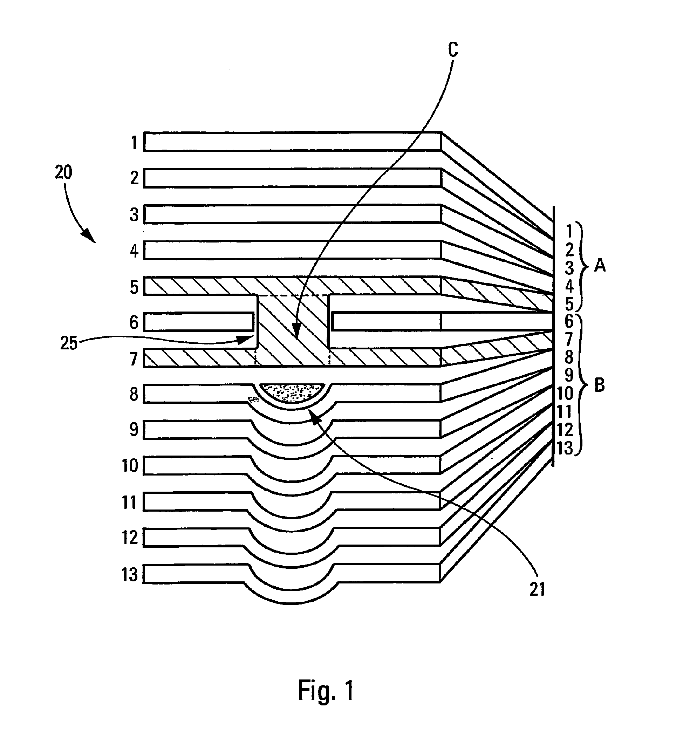

L'exemple de réalisation représenté sur la figure 1 montre une structure de bande de blisters comportant treize couches différentes. Il est toutefois entendu qu'une partie de ces couches n'est qu'optionnelle, comme cela sera explicité plus précisément ci-après.

La bande de blisters 20 de l'invention comporte une pluralité de blisters formés chacun par un réservoir 21 comportant une ouverture 25. La bande de blister 20 comporte au moins une couche de base 6 qui est pourvue des ouvertures 25 formant les ouvertures des blisters. Une couche de cavités 8 est prévue, ladite couche de cavités étant pourvue de cavités 21 formant les parois des blisters. Enfin une couche déchirable est prévue pour obturer de manière étanche chaque blister, ladite couche déchirable étant ouverte lorsque l'utilisateur souhaite distribuer la dose de produit contenue dans le blister. De préférence, ce produit est une poudre sèche, notamment, une poudre pharmaceutique.

Selon l'invention, la couche déchirable comporte une première partie de couche déchirable 7 qui est disposée entre ladite couche de base 6 et ladite couche de cavités 8. Par ailleurs, la couche déchirable comporte une seconde partie de couche déchirable 5 disposée du côté opposé de ladite couche de base 6. Au niveau de chaque ouverture 25 de la couche de base 6, lesdites première et seconde parties de couche déchirable 7, 5 sont reliées l'une à l'autre. De préférence, cette liaison est réalisée de manière monobloc, avantageusement par fusion de matière, et il est alors préféré de réaliser les première et seconde parties de couche déchirable avec le même matériau. Avantageusement, cette couche déchirable peut comprendre du polyéthylène. Avantageusement, les première et seconde parties de couche déchirable 7, 5 sont chacune constituées d'un film continu, notamment de polyéthylène, par exemple extrudé, qui est appliqué sur la couche de base et dont l'épaisseur peut être inférieure à 100µm, avantageusement comprise entre 10µm et 40µm, de préférence 30µm. Comme cela est représenté schématiquement sur la figure 1, lors de cette application, il se créé une fusion de matière entre les premières et secondes parties de couche déchirable au niveau des ouvertures 25 de cette couche de base 6, garantissant une étanchéité absolue du contenu du blister 21 en position de fermeture. La couche de base 6 comprend avantageusement du polyester et peut avoir une épaisseur inférieur à 100µm, avantageusement comprise entre 40µm et 60µm, de préférence 50µm. Par ailleurs, la couche de cavités 8 peut comprendre du polyéthylène et/ou du polypropylène, et peut avoir une rigidité légèrement supérieure pour permettre de réaliser les cavités 21 formant les blisters.

Comme représenté sur la figure 1, diverses couches optionnelles peuvent être prévues dans la structure multicouche de la bande de blisters 20 de l'invention. Ainsi, la couche déchirable peut en outre comprendre une première couche d'aluminium 2, d'épaisseur inférieure à 50µm, avantageusement comprise entre 10µm et 30µm, de préférence 20µm, et destinée principalement à protéger le contenu du blister de l'humidité. Cette feuille d'aluminium 2 est avantageusement fixée à la couche déchirable, notamment à la seconde partie de couche déchirable 5, avec interposition d'un film de polyester et d'une couche d'adhésif. Bien entendu, on pourrait envisager d'autres moyens de fixation de la couche d'aluminium 2 à la couche déchirable7, 5. La couche de polyester 4 peut être réalisée sous la forme d'un film ayant une épaisseur inférieure à 50 µm, avantageusement comprise entre et 10 et 15µm, de préférence 12µm, et l'adhésif peut être une colle quelconque appropriée. Avantageusement, on prévoit en outre une première couche externe 1, qui peut être avantageusement formée par une laque d'impression, facilitant l'impression d'inscription sur la couche déchirable. D'autre part, la couche de cavités 8 peut également comporter une seconde couche d'aluminium 11, également destinée principalement à la protection contre l'humidité. De manière similaire, cette seconde couche d'aluminium 11 peut être fixée à la couche de cavités 8 avec interposition d'une couche de polyester 9 et d'une couche d'adhésif approprié 10. De même, une seconde couche externe 13 peut être prévue du côté de la couche de cavités 8, de préférence formée par une couche de protection ou de vernis, et cette seconde couche externe 13 peut être fixée au moyen d'une couche d'adhésif 12. L'interposition des couches de polyester 4 et 9 peut faciliter la fixation des couches d'aluminium 2 et 11 de chaque côté de la bande de blisters 20.

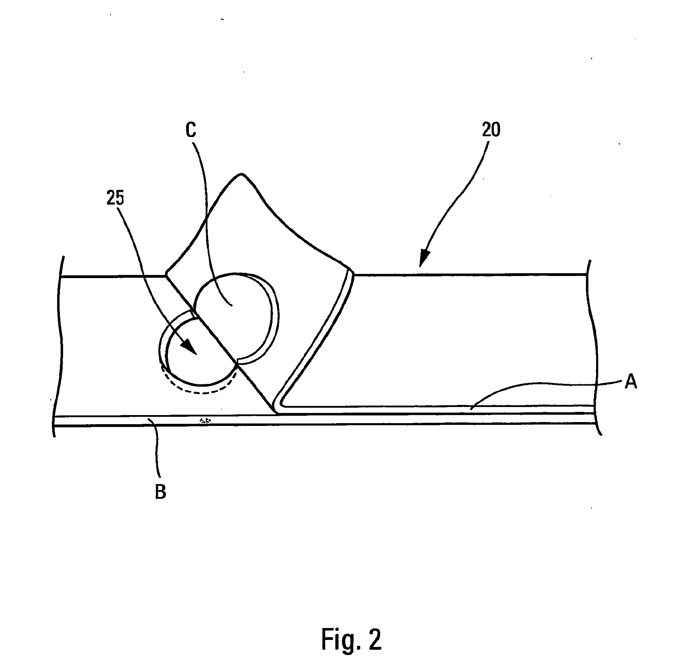

En se référent à la figure 2, qui montre un processus d'ouverture d'une bande de blister 20 telle que décrite précédemment, on constate que cette structure multicouche n'est formée en fait après assemblage, que de deux parties séparables, une partie de cavités, constituée des couches 6 à 13 décrite sur la figure 1, et une partie de bande déchirable A constituée des couches 1 à 5 décrite sur la figure 1. Bien entendu, comme expliqué précédemment, la structure multicouche peut être réalisée de manière différente à celle décrite en référence à l'exemple de la figure 1.

En fonctionnement, lorsqu'un blister 21 de la bande à blisters 20 doit être ouvert, une traction sur la partie de bande A aura pour effet de soulever cette bande A à partir de la partie de cavités B. Lorsque l'on atteint une ouverture 25, le bord de chaque ouverture 25 de la couche de base 6 déchire la liaison de matière réalisée entre les première et seconde parties de couche déchirable 7, 5 de sorte qu'un disque C de la première partie de couche déchirable 7 reste fixé à la seconde partie de couche déchirable 5 et donc à la bande déchirable A, ouvrant ainsi la cavité du blister 21 pour libérer le contenu de celle-ci. La présence d'une partie de couche déchirable de chaque côté de la bande de base 6 garantit une ouverture nette est précise du blister 25 et évite notamment toute présence de bavure ou de partie de couche déchirable résiduelle sur les bords de l'ouverture qui risquerait de retenir une partie du produit contenu dans la cavité de blisters. La distribution de la totalité de la dose est donc garantie avec la bande de blisters de l'invention. De même, la bande de blisters de l'invention est particulièrement adaptée à être utilisée dans un inhalateur, notamment du type comportant un système d'ouverture de blisters actionné par l'inhalation. En effet, la présente invention permet de réaliser la couche déchirable 7, 5 avec une adhérence à la couche de base 6 entre les ouvertures 25 qui peut être différente, notamment inférieure par rapport à l'adhérence de la couche déchirable 7, 5 à la couche de base 6 à proximité ou au niveau desdites ouvertures. L'effort d'ouverture de chaque dose peut donc être adapté de manière optimale, tout en garantissant une étanchéité absolue de par la liaison de matière entre les deux parties de couches déchirables. On peut de ce fait réaliser une bande de blisters qui peut être aisément et sûrement ouverte au moyen d'un système déclenché par l'inhalation de l'utilisateur.

Les matériaux décrits en référence à la structure représentée sur la figure 1 peuvent également être modifiées, en utilisant notamment des matériaux similaires ou ayant des propriétés appropriées. En particulier, la couche de base, la couche de cavités et la couche déchirable peuvent être réalisées en tous matériaux appropriés dans ce but par exemple de la famille des polyoléfines. De même, les épaisseurs sont données à titre indicatif et pourraient être réalisées de manière différente. De plus, l'assemblage des différentes couches les une par rapport aux autres peut aussi être réalisé d'une manière quelconque appropriée.

D'autres modifications sont également envisageables pour un homme du métier sans sortir du cadre de la présente invention telle que défini par les revendications annexées.

## Revendications

1. Bande de blisters (20) destinée à être utilisée dans un inhalateur de produit fluide ou pulvérulent, comportant une pluralité de blisters formés chacun par un réservoir (21) comportant une ouverture (25) obturée de manière étanche par une couche déchirable (7, 5), la bande de blisters (20) comportant au moins une couche de base (6) pourvue d'ouvertures (25) formant les ouvertures des blisters, et une couche de cavités (8) pourvue de cavités formant les parois de blisters (21), **caractérisée en ce que** ladite couche déchirable (7, 5) comporte une première partie de couche déchirable (7) disposée entre ladite couche de base (6) et ladite couche de cavités (8), et une seconde partie de couche déchirable (5) disposée du côté opposé de ladite couche de base (6), lesdites première et seconde parties de couche déchirable (7, 5) étant reliées l'une à l'autre au niveau de chaque ouverture (25) de la couche de base (6).

2. Bande de blisters selon la revendication 1, dans laquelle lesdites première et seconde parties de couche déchirable (7, 5) sont réalisées avec le même matériau.

3. Bande de blisters selon la revendication 1 ou 2, dans laquelle lesdites première et seconde parties de couche déchirable (7,5) sont reliées l'une à l'autre de manière monobloc au niveau de chaque ouverture (25) de la couche de base (6).

4. Bande de blisters selon la revendication 3, dans laquelle ladite liaison monobloc est réalisée par fusion de matière.

5. Bande de blisters selon l'une quelconque des revendications précédentes, dans laquelle ladite couche déchirable (7,5) comprend du polyéthylène.

6. Bande de blisters selon l'une quelconque des revendications précédentes, dans laquelle lesdites première et seconde parties de couche déchirable (7, 5) sont chacune constituées d'un film continu de polyéthylène.

7. Bande de blisters selon l'une quelconque des revendications précédentes,
dans laquelle lesdites première et seconde parties de couche déchirable (7, 5) comportent chacune un film ayant une épaisseur inférieure à 100µm, avantageusement comprise entre 10µm et 40µm, de préférence 30µm.

8. Bande de blisters selon l'une quelconque des revendications précédentes, dans laquelle ladite couche de base (6) comprend du polyester.

9. Bande de blisters selon l'une quelconque des revendications précédentes, dans laquelle ladite couche de base (6) comprend un film ayant une épaisseur inférieure à 100µm, avantageusement comprise entre 40µm et 60µm, de préférence 50µm.

10. Bande de blisters selon l'une quelconque des revendications précédentes, dans laquelle ladite couche de cavités (8) comprend du polyéthylène et/ou du polypropylène.

11. Bande de blisters selon l'une quelconque des revendications précédentes, dans laquelle ladite couche déchirable (7, 5) comporte en outre une première couche d'aluminium (2) fixée à ladite seconde partie de couche déchirable (5).

12. Bande de blisters selon la revendication 11, dans laquelle ladite première couche d'aluminium (2) a une épaisseur inférieur à 50µm, avantageusement comprise entre 10µm et 30µm, de préférence 20µm.

13. Bande de blisters selon la revendication 11 ou 12, dans laquelle une couche de polyester (4) et une couche d'adhésif (3) sont disposées entre ladite seconde partie de couche déchirable (5) et ladite première couche d'aluminium (2).

14. Bande de blisters selon l'une quelconque des revendications précédentes, dans laquelle ladite couche déchirable (7, 5) comporte une première couche externe (1), de préférence formée par une laque d'impression.

15. Bande de blisters selon l'une quelconque des revendications précédentes, dans laquelle ladite couche de cavités (8) comporte en outre une seconde couche d'aluminium (11).

16. Bande de blisters selon la revendication 15, dans laquelle une couche de polyester (9) et une couche d'adhésif (10) sont disposées entre ladite couche de cavités (8) et ladite seconde couche d'aluminium (11).

17. Bande de blisters selon l'une quelconque des revendications précédentes, dans laquelle ladite couche de cavités (8) comporte une seconde couche externe (13), de préférence formée par une couche de protection ou une couche de vernie, de préférence avec interposition d'une couche d'adhésif (12).

18. Bande de blisters selon l'une quelconque des revendications précédentes, dans laquelle l'adhérence de la couche déchirable (7, 5) à la couche de base (6) entre les ouvertures (25) est différente de l'adhérence à proximité desdites ouvertures (25).

19. Bande de blisters selon l'une quelconque des revendications précédentes, dans laquelle les blisters (21) contiennent une poudre pharmaceutique.

20. Inhalateur de poudre sèche, **caractérisé en ce qu'**il comporte une bande de blisters (20) selon l'une quelconque des revendications précédentes.

## Claims

1. A blister strip (20) for use in a fluid or powder inhaler, and including a plurality of blisters, each formed by a reservoir (21) including an opening (25) that is sealed in leaktight manner by a tearable layer (7, 5), the blister strip (20) comprising at least a base layer (6) that is provided with openings (25) forming the openings of the blisters, and a cavity layer (8) that is provided with cavities forming the blister walls (21), **characterized in that** said tearable layer (7, 5) comprises a first tearable-layer portion (7) that is disposed between said base layer (6) and said cavity layer (8), and a second tearable-layer portion (5) that is disposed on the opposite side of said base layer (6), said first and second tearable-layer portions (7, 5) being connected together at each opening (25) of the base portion (6).

2. A blister strip according to claim 1, in which said first and second tearable-layer portions (7, 5) are made from the same material.

3. A blister strip according to claim 1 or claim 2, in which said first and second tearable-layer portions (7, 5) are connected together as a single part in each opening (25) of the base layer (6).

4. A blister strip according to claim 3, in which said single-part connection is made by fusing material.

5. A blister strip according to any preceding claim, in which said tearable layer (7, 5) comprises polyethylene.

6. A blister strip according to any preceding claim, in which each of said first and second tearable-layer portions (7, 5) are constituted by a continuous film of polyethylene.

7. A blister strip according to any preceding claim, in which each of said first and second tearable-layer portions comprises a film having thickness that is less than 100 µm, advantageously lying in the range 10 µm to 40 µm, and preferably equal to 30 µm.

8. A blister strip according to any preceding claim, in which said base layer (6) comprises polyester.

9. A blister strip according to any preceding claim, in which said base layer (6) comprises a film having thickness that is less than 100 µm, advantageously lying in the range 40 µm to 60 µm, and preferably equal to 50 µm.

10. A blister strip according to any preceding claim, in which said cavity layer (8) comprises polyethylene and/or polypropylene.

11. A blister strip according to any preceding claim, in which said tearable layer (7, 5) further includes a first aluminum layer (2) that is fastened to said second tearable-layer portion (5).

12. A blister strip according to claim 11, in which said first aluminum layer (2) has thickness that is less than 50 µm, advantageously lying in the range 10 µm to 30 µm, and preferably equal to 20 µm.

13. A blister strip according to claim 11 or claim 12, in which a polyester layer (4) and an adhesive layer (3) are disposed between said second tearable-layer portion (5) and said first aluminum layer (2).

14. A blister strip according to any preceding claim, in which said tearable layer (7, 5) includes a first outer layer (1), preferably formed by a printers' varnish.

15. A blister strip according to any preceding claim, in which said cavity layer (8) further includes a second aluminum layer (11).

16. A blister strip according to claim 15, in which a polyester layer (9) and an adhesive layer (10) are disposed between said cavity layer (8) and said second aluminum layer (11).

17. A blister strip according to any preceding claim, in which said cavity layer (8) includes a second outer layer (13), preferably formed by a protective layer or by a layer of varnish, preferably interposed with an adhesive layer (12).

18. A blister strip according to any preceding claim, in which the adherence of the tearable layer (7, 5) to the base layer (6) between the openings (25) is different from the adherence in the proximity of said openings (25).

19. A blister strip according to any preceding claim, in which the blisters (21) contain a pharmaceutical powder.

20. A dry-powder inhaler, **characterized in that** it includes a blister strip (20) according to any preceding claim.

## Patentansprüche

1. Blisterstreifen (20), der dazu bestimmt ist, in einer Inhalationsvorrichtung für ein fluidförmiges oder pulverförmiges Produkt verwendet zu werden und der eine Vielzahl von Blistern umfasst, von denen jeder von einem Behälter (21) gebildet wird, der eine in dichter Weise durch eine abreißbare Schicht (7, 5) verschlossene Öffnung (25) aufweist, wobei der Blisterstreifen (20) wenigstens eine Basisschicht (6) umfasst, die mit Öffnungen (25) versehen ist, welche die Öffnungen der Blister bilden, sowie eine Hohlraumschicht (8), die mit Hohlräumen versehen ist, welche die Wände (21) der Blister bilden, **dadurch gekennzeichnet, dass** die ablösbare Schicht (7, 5) einen ersten Teil (7) der ablösbaren Schicht, der zwischen der Basisschicht (6) und der Hohlraumschicht (8) angeordnet ist, und einen zweiten Teil (5) der ablösbaren Schicht umfasst, der auf der dieser Basisschicht (6) gegenüberliegenden Seite angeordnet ist, wobei der erste und der zweite Teil der ablösbaren Schicht (7, 5) im Bereich einer jeden Öffnung (25) der Basisschicht (6) miteinander verbunden sind.

2. Blisterstreifen nach Anspruch 1, bei dem der erste und der zweite Teil der ablösbaren Schicht (7, 5) aus dem gleichen Material hergestellt sind.

3. Blisterstreifen nach Anspruch 1 oder 2, bei dem der erste und der zweite Teil der ablösbaren Schicht (7, 5) im Bereich einer jeden Öffnung (25) der Basisschicht (6) miteinander einstückig verbunden sind.

4. Blisterstreifen nach Anspruch 3, bei dem die einstückige Verbindung durch Schmelzen des Materials realisiert ist.

5. Blisterstreifen nach einem der vorhergehenden Ansprüche, bei dem die ablösbare Schicht (7, 5) Polyethylen umfasst.

6. Blisterstreifen nach einem der vorhergehenden Ansprüche, bei dem der erste und der zweite Teil der ablösbaren Schicht (7, 5) jeweils von einem kontinuierlichen Film aus Polyethylen gebildet werden.

7. Blisterstreifen nach einem der vorhergehenden Ansprüche, bei dem der erste und der zweite Teil der ablösbaren Schicht (7, 5) jeweils einen Film umfassen, der eine Dicke von weniger als 100 µm, in vorteilhafter Weise zwischen 10 µm und 40 µm und in bevorzugter Weise gleich 30 µm besitzt.

8. Blisterstreifen nach einem der vorhergehenden Ansprüche, bei dem die Basisschicht (6) Polyester umfasst.

9. Blisterstreifen nach einem der vorhergehenden Ansprüche, bei dem die Basisschicht (6) einen Film umfasst, der eine Dicke von weniger als 100 µm, in vorteilhafter Weise zwischen 40 µm und 60 µm und in bevorzugter Weise gleich 50 µm besitzt.

10. Blisterstreifen nach einem der vorhergehenden Ansprüche, bei dem die Hohlraumschicht (8) Polyethylen und/oder Polypropylen umfasst.

11. Blisterstreifen nach einem der vorhergehenden Ansprüche, bei dem die ablösbare Schicht (7, 5) weiterhin eine erste Aluminiumschicht (2) umfasst, die an dem zweiten Teil (5) der ablösbaren Schicht befestigt ist.

12. Blisterstreifen nach Anspruch 11, bei dem die erste Aluminiumschicht (2) eine Dicke von weniger als 50 µm, i vorteilhafter Weise zwischen 10 µm und 30 µm und in bevorzugter Weise gleich 20 µm besitzt.

13. Blisterstreifen nach Anspruch 11 oder 12, bei dem eine Polyesterschicht (4) und eine Klebstoffschicht (3) zwischen dem zweiten Teil (5) der ablösbaren Schicht und der ersten Aluminiumschicht (2) angeordnet sind.

14. Blisterstreifen nach einem der vorhergehenden Ansprüche, bei dem die ablösbare Schicht (7, 5) eine erste Außenschicht (1) umfasst, die vorzugsweise von einem Drucklack gebildet wird.

15. Blisterstreifen nach einem der vorhergehenden Ansprüche, bei dem die Hohlraumschicht (8) weiterhin eine zweite Aluminiumschicht (11) umfasst.

16. Blisterstreifen nach Anspruch 15, bei dem eine Polyesterschicht (9) und eine Klebstoffschicht (10) zwischen der Hohlraumschicht (8) und der zweiten Aluminiumschicht (11) angeordnet sind.

17. Blisterstreifen nach einem der vorhergehenden Ansprüche, bei dem die Hohlraumschicht (8) eine zweite Außenschicht (13) umfasst, die vorzugsweise von einer Schutzschicht oder Lackschicht gebildet wird, wobei vorzugsweise eine Klebstoffschicht (12) dazwischen angeordnet ist.

18. Blisterstreifen nach einem der vorhergehenden Ansprüche, bei dem das Anhaften der ablösbaren Schicht (7, 5) an der Basisschicht (6) zwischen den Öffnungen (25) vom Anhaften in der Nähe dieser Öffnungen (25) verschieden ist.

19. Blisterstreifen nach einem der vorhergehenden Ansprüche, bei dem die Blister (21) ein pharmazeutisches Pulver enthalten.

20. Inhalationsvorrichtung für ein trockenes Pulver, **dadurch gekennzeichnet, dass** sie einen Blisterstreifen (20) nach einem der vorhergehenden Ansprüche umfasst.
